(19) Europäisches Patentamt
European Patent Office
Office européen des brevets

(11) **EP 3 256 597 B1**

(12) **EUROPEAN PATENT SPECIFICATION**

(45) Date of publication and mention of the grant of the patent:
**14.11.2018 Bulletin 2018/46**

(21) Application number: **16703769.6**

(22) Date of filing: **09.02.2016**

(51) Int Cl.:
***C12P 21/00*** *(2006.01)* ***C12P 21/02*** *(2006.01)*
***C12P 21/06*** *(2006.01)* ***C12N 9/50*** *(2006.01)*

(86) International application number:
**PCT/EP2016/052658**

(87) International publication number:
**WO 2016/128363 (18.08.2016 Gazette 2016/33)**

(54) **METHOD FOR PRODUCING A RECOMBINANT PROTEIN OF INTEREST**

VERFAHREN ZUR HERSTELLUNG EINES REKOMBINANTEN PROTEINS VON INTERESSE

PROCÉDÉ DE PRODUCTION D'UNE PROTÉINE RECOMBINANTE D'INTÉRÊT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.02.2015 EP 15154320**

(43) Date of publication of application:
**20.12.2017 Bulletin 2017/51**

(73) Proprietor: **Sandoz AG**
**4056 Basel (CH)**

(72) Inventors:
- **KELLER, Sascha**
**6250 Kundl/Tirol (AT)**
- **JASIAK, Anna Justyna**
**6250 Kundl/Tirol (AT)**
- **FUNKE, René**
**6250 Kundl/Tirol (AT)**

(74) Representative: **Sonn & Partner Patentanwälte**
**Riemergasse 14**
**1010 Wien (AT)**

(56) References cited:
**EP-A1- 2 684 951 EP-A1- 2 746 390**

- **ASTRID DÜRAUERAB ET AL: "Npro Autoprotease Fusion Technology: Development, Characteristics, and Influential Factors", SEPARATION SCIENCE AND TECHNOLOGY, DEKKER, NEW YORK, NY, US, vol. 45, no. 15, 1 January 2010 (2010-01-01), pages 2194-2209, XP009166982, ISSN: 0149-6395, DOI: 10.1080/15228967.2010.507552**
- **JUNGBAUER ET AL: "Current status of technical protein refolding", JOURNAL OF BIOTECHNOLOGY, ELSEVIER SCIENCE PUBLISHERS, AMSTERDAM, NL, vol. 128, no. 3, 24 January 2007 (2007-01-24), pages 587-596, XP005856755, ISSN: 0168-1656, DOI: 10.1016/J.JBIOTEC.2006.12.004**
- **CLEMENS ACHMÜLLER ET AL: "N-pro fusion technology to produce proteins with authentic N termini in E-coli", NATURE METHODS, NATURE PUBLISHING GROUP, GB, vol. 4, no. 12, 1 December 2007 (2007-12-01), pages 1037-1043, XP002663733, ISSN: 1548-7091, DOI: 10.1038/NMETH1116 [retrieved on 2007-11-18] cited in the application**
- **WALTRAUD KAAR ET AL: "Refolding of N pro fusion proteins", BIOTECHNOLOGY AND BIOENGINEERING, vol. 104, no. 4, 1 November 2009 (2009-11-01), pages n/a-n/a, XP055050976, ISSN: 0006-3592, DOI: 10.1002/bit.22432**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 256 597 B1

- **KUDOU M ET AL: "A novel protein refolding system using lauroyl-l-glutamate as a solubilizing detergent and arginine as a folding assisting agent", PROTEIN EXPRESSION AND PURIFICATION, ACADEMIC PRESS, SAN DIEGO, CA, vol. 75, no. 1, 1 January 2011 (2011-01-01), pages 46-54, XP027445017, ISSN: 1046-5928, DOI: 10.1016/J.PEP.2010.08.011 [retrieved on 2010-09-09]**
- **BENJAMIN SCHLAGER ET AL: "Use of anionic denaturing detergents to purify insoluble proteins after overexpression", BMC BIOTECHNOLOGY, BIOMED CENTRAL LTD. LONDON, GB, vol. 12, no. 1, 11 December 2012 (2012-12-11), page 95, XP021135820, ISSN: 1472-6750, DOI: 10.1186/1472-6750-12-95**

SEQUENCE LISTING

<110> Sandoz AG

<120> Method for producing a recombinant protein of interest

<130> R 68562

<150> EP 15154320.4
<151> 09.02.2015

<160> 1

<170> PatentIn version 3.5

<210> 1
<211> 16
<212> PRT
<213> Artificial Sequence

<220>
<223> psp6his model peptide

<400> 1

```
Ser Val Asp Lys Leu Ala Ala Ala Leu Glu His His His His His His
1               5                   10                  15
```

## Description

The present invention relates to a process for the recombinant production of a desired heterologous polypeptide of interest by using the autoprotease $N^{pro}$ of Pestivirus-technology.

Overexpression of heterologous proteins in E. coli frequently leads to aggregation and deposition in dense, insoluble particles, also known as inclusion bodies. Advantages of the expression in inclusion bodies are the high purity of the desired product and the easy purification by centrifugation after cell disruption. However, crucial steps are resolving and refolding of the protein into its native structure. Solubilisation usually is carried out in high concentrations of chaotropic agents like urea or guanidinium chloride (guanidine hydrochloride) to reach complete unfolding. Reducing agents such as 2-mercaptoethanol (β-ME), dithiothreitol (DTT), dithioerythritol (DTE) or 1-monothioglycerol (MTG) are added to reduce non-native inter- and intramolecular disulfide bonds and keep the cysteins in a reduced state.

A bottleneck step is the renaturation of the proteins. Elimination of hydrophobic intermolecular interaction during the first steps of refolding is crucial for successful renaturation at high protein concentrations and to prevent aggregation (Vallejo et al., Microb. Cell Fact. 3 (2004), 11). Several renaturation techniques are known.

A technology platform was established by using the genetically engineered $N^{pro}$ autoprotease from classical swine fever virus (CSFV) to produce difficult-to-express therapeutic peptides and proteins in form of inclusion bodies in E. coli. This fusion protein technology processing requires renaturation of the inclusion bodies, autoprotease cleavage and refolding of the released target molecule which is usually performed in batch mode. Due to its simplicity refolding by dilution is preferred to pressure treatment or chromatographic techniques, especially in production scale. Protein concentration, as well as chaotrop concentration is diminished in a single step preventing aggregation by intermolecular interactions. However, large volumes and low protein concentration burden downstream processing steps. (Jungbauer et al., J. Biotech. 128 (2007), 587-596) . EP2746390-A1 discloses a method for producing Npro fusion protein in inclusion bodies and using chaotropic conditions for solubilization. Duraurab et al., Separation Science and technology (2010), 2194-2209, discloses a similar method and points to the benefits of kosmotropic conditions for the Npro activity.

It is therefore an object of the present invention to provide an improvement in renaturation of inclusion bodies which must be renaturated, especially for autoproteolytic cleavage and preparation of recombinant proteins downstream of the process. Preferably, the invention should enable low process volumes and high protein concentrations for obtaining the protein of interest and provide a method which is suitable to be established in industrial production scale, specifically for proteins used in medicine.

Therefore, the present invention provides a method for producing a recombinant protein of interest, characterised in by the following steps:

(a) providing a fusion protein comprising an $N^{pro}$ autoprotease moiety and a protein of interest moiety in inclusion bodies,
(b) solubilising the fusion protein in the inclusion bodies by subjecting the inclusion bodies to a solubilisation buffer containing a detergent and wherein the solubilisation buffer contains no chaotropes or chaotropes in a concentration corresponding to less than 1.5 M urea, wherein the detergent in the solubilisation buffer is a lauroyl amino acid, especially lauroyl-L-glutamate, lauroyl-sarcosinate, or mixtures thereof;
(c) refolding the solubilised fusion protein and
(d) allowing the fusion protein to be cleaved by the $N^{pro}$ autoprotease moiety under kosmotropic conditions, wherein the recombinant protein of interest is cleaved from the fusion protein, and
(e) recovering the protein of interest.

The present invention is an improvement in the recombinant production of a desired heterologous polypeptide of interest by using the autoprotease $N^{pro}$ of Pestivirus-technology. This technology usually provides the recombinant expression of a fusion polypeptide which comprises an autoproteolytic moiety directly or indirectly derived from autoprotease $N^{pro}$ of Pestivirus and a heterologous polypeptide of interest in a host cell, often a prokaryotic host cell, such as E. coli. The heterologous polypeptide or protein of interest is covalently coupled via a peptide bond to the $N^{pro}$ molecule. The protein of interest is released from the fusion protein through hydrolysis of the peptide bond between the C-terminal Cys168 of $N^{pro}$ and position 169 of the fusion polypeptide which represents the authentic N-terminal amino acid of the protein of interest to be produced according to the present invention. The heterologous polypeptide of interest is produced in the host cell in form of cytoplasmic inclusion bodies (IB), which are then isolated and treated in such a way, that the desired heterologous polypeptide is cleaved from the fusion polypeptide by the $N^{pro}$ autoproteolytic activity.

Fusion polypeptides comprising the autoprotease $N^{pro}$ of Pestivirus are therefore specifically useful for producing heterologous recombinant polypeptides. $N^{pro}$ is an autoprotease with length of 168 amino acids and an apparent $M_r$ of about 20 kD in vivo. It is the first protein in the polyprotein of Pestiviruses and undergoes autoproteolytic cleavage from the following nucleocapsid protein C. This cleavage takes place after the last amino acid in the sequence of $N^{pro}$, Cys168. The autoprotease $N^{pro}$ activity of Pestivirus always cleaves off the fusion partner at this clearly determined site, releasing a polypeptide of interest with homogenous N-terminus. In addition, the autoproteolytic activity of $N^{pro}$ can be induced in vitro, by application of special buffers, so that the polypeptide of interest can be obtained by cleavage of fusion polypeptides that are expressed in IBs.

N-terminal autoprotease $N^{pro}$ from Classical Swine Fever Virus (CSFV) used in this technology serves as an attractive tool for expression of therapeutic proteins in large amounts especially in E. coli. Medical applications require an authentic N-terminus of the recombinant proteins, which can be achieved by self-cleavage of N-terminally fused $N^{pro}$ autoprotease. In addition, $N^{pro}$ fusion technology also allows the expression of small or toxic peptides, which would be degraded immediately after their synthesis by host cell proteases (Achmuller et al., Nat. Methods 4 (2007), 1037-1043). As the expression of $N^{pro}$ fusion proteins in E. coli leads to the formation of insoluble aggregates, known as inclusion bodies, appropriate resolving and renaturation protocols are required to obtain biological active proteins.

As already mentioned, in most cases solubilisation is carried out in chaotropic agents such as urea or guanidinium chloride at high concentrations in combination with reducing agents to abolish false formed disulfide bonds. Due to its simplicity refolding by dilution is widely used to initiate renaturation. Hence, large amounts of buffer are added to provide conditions, which allow the formation of the correct biological active structure.

In contrast thereto, the present invention allows the significant reduction of renaturing buffer due to the presence of low amounts of detergents in the solubilisation buffer. Surprisingly, this also leads to a significantly improved renaturation yield and allows shorter process duration. In this connection it has to be noted that the reduction of the refolding buffer volume is limited by the solubility of the folding aids (e.g. sugars and amino acids). Since in the practical embodiments of this technology it is usually worked near the edge of solubility, a further reduction of only a small buffer volume is still advantageous.

The present invention also allows renaturation with higher concentration of fusion proteins leading to a lowering of process volumes. The detergents used can easily be removed from the fusion protein or the final protein of interest by standard procedures for removal of detergents (or by the steps previously foreseen to eliminate the denaturing agents (chaotropes, such as urea or guanidinium chloride)). Preferably, the detergent is removed from the fusion protein or the final protein of interest by chromatographic methods, for example ion exchange chromatography.

With the present invention, significant amount of chaotrope material can be replaced by the addition of detergents. This also leads to a lower viscosity of the process buffers which saves energy especially in industrial large scale use of the present invention. Also the total amount of buffer needed in the process is reduced which leads to lower costs for raw material and waste disposal.

Solubilisation according to the present invention will occur at detergent concentrations greater than the CMC (critical micelle concentration). This implies that refolding will usually start after dilution below the CMC (however, refold may also occur above the CMC); refolding results (for $N^{pro}$ autocatalytic activity) in cleavage of the fusion protein. Micelles are required in order to solubilize the IBs in aqueous solutions. Therefore the CMC (which is i.a. temperature dependent) is one the most important detergent characteristics according to the present invention. Below the CMC refolding could theoretically start - but most likely no solubilisation or rapid precipitation (after dilution) will occur. In solubilisation condition no cleavage is observed. In this connection it can also be mentioned that the supportive effect of detergents according to the present invention on $N^{pro}$ fusion protein refolding/cleavage is not general but dependent on the individual detergent properties.

Accordingly, in a preferred method the inclusion bodies were generated in a recombinant production system, preferably in a prokaryotic host cell, especially in E. coli host cells.

According to a preferred embodiment, the detergent is contained in the solubilisation buffer in a concentration of 0.2 to 15 % (w/v), preferably of 0.3 to 10 % (w/v), especially of 0.4 to 5 % (w/v). Preferred concentrations of the detergents used in the present invention are defined according to the individual CMC of a given detergent. For solubilisation, a concentration above the CMC has to be applied (Ariki et al., J. Biochem. 151 (2012), 27-33). In the course of the present invention that - at least for some detergents - renaturation yields reached a maximum slightly above the critical micelle concentration. For effecting cleavage, the detergent concentration has to be brought near the CMC.

Preferably, the detergent is removed for cleavage to a certain extent. Since complete removal would cause precipitation, a small amount of detergent is required for the refolding. However, the refolding yield is increased, if the target protein concentration is lowered - therefore, a dilution is preferably performed. Lowering detergent concentrations or removal of detergents can be effected by various means, such as dilution, extraction, precipitation, binding to solid surfaces (chromatographic material, especially cation exchange chromatography)), etc.. This can be achieved gradually or in a single step. Both mechanisms - refolding and cleavage - occur almost in parallel. The renaturation (= refolding) itself is started with the reduction of detergent below CMC.

The detergents for use in the solubilisation buffer are lauroyl amino acids, especially lauroyl-L-glutamate ("NLG"), lauroyl-sarcosinate ("NLS"), or mixtures thereof.

For each of the detergents, optimised conditions can be chosen based on the physiochemical properties such as CMC (or charge), i.e. parameters that are known for each of the detergents. For example, the CMC for NLS is 14.57 mM (30°C) or 15.25 (25°C) (corresponding to 414mg/L (MW = 271.40 g/mol)); CMC for NLG is 4.84 mM (25°C) (corresponding to 0.17 g/L (MW = 351.41 g/mol)). Above and below CMC the effect of solubilisation and refolding occurs, respectively. Accordingly, e.g. concentrations of 0.5 to 5 % (w/v), preferably 0.7 to 4 % (w/v), especially 1 to 3 % (w/v), are preferred in the solubilisation buffer for anionic detergents, especially NLS/NLG. In the renaturation buffer, the preferred detergent concentrations are in this example (anionic detergents, especially NLS/NLG) 0 to 0.35 % (w/v), preferably 0 to 0.3 % (w/v), especially 0 to 0.2 % (w/v) .

Some detergents are preferably used alone (i.e. without the presence of chaotropes during solubilisation); some have improved solubilisation characteristics if they are combined with chaotropes during solubilisation (it is also possible to improve solubilisation and renaturation yields in cases where very low detergent concentrations are applied by the addition of chaotropes; however, the major aim of the present invention is to work with the lowest chaotrope concentration possible, preferably to exclude chaotropes completely during solubilisation). For example, anionic detergents, such as NLS and NLG, are able to solubilise IBs without the need for presence of chaotropes (in fact, usually yields are lowered if chaotropes are present).

Preferably, steps (c) and/or (d) are performed at kosmotropic conditions that correspond to a urea concentration of below 1.5 M urea (where no solubilisation effect takes place), e.g. urea concentrations from 0 to 1.5 M, preferably from 0.2 to 1 M, especially from 0.4 to 0.8 M.

With the present invention, the solubilisation buffer can either be free of chaotropic substances (which is preferred), or at least be provided with a chaotrope concentration of less than 1.5 M (whereas "classical" solubilisation buffers contained 5 M urea or more or 3 M guanidine hydrochloride. Accordingly, chaotropes can either be completely removed from the solubilisation buffer or be provided at a concentration of below 1.5 M urea (or another chaotrope in a concentration corresponding to below 1.5 M urea; "correspond to" means that either urea is present in the amount indicated or that another chaotropic substance (such as butanol, ethanol, guanidinium chloride, lithium perchlorate, magnesium chloride, phenol, propanol, sodium dodecyl sulfate, thiourea, etc.) is present in a concentration which leads to the same chaotropic effect (measured as increase of the entropy of the system)).

The terms "kosmotrope" (order-maker) and "chaotrope" (disorder-maker) originally denoted solutes that stabilized, or destabilized respectively, proteins and membranes. Later they referred to the apparently correlating property of increasing, or decreasing respectively, the structuring of water. Such properties may vary dependent on the circumstances, method of determination or the solvation shell(s) investigated. An alternative term used for kosmotrope is "compensatory solute" as they have been found to compensate for the deleterious effects of high salt contents (which destroy the natural hydrogen bonded network of water) in osmotically stressed cells. Both the extent and strength of hydrogen bonding may be changed independently by the solute but either of these may be, and has been, used as measures of order making. It is, however, the effects on the extent of quality hydrogen bonding that is of overriding importance. The ordering effects of kosmotropes may be confused by their diffusional rotation, which creates more extensive disorganized junction zones of greater disorder with the surrounding bulk water than less hydrated chaotropes. Most kosmotropes do not cause a large scale net structuring in water.

Ionic kosmotropes (or: "antichaotropes" to distinguish them from non-ionic kosmotropes) should be treated differently from non-ionic kosmotropes, due mainly to the directed and polarized arrangements of the surrounding water molecules. Generally, ionic behaviour parallels the Hofmeister series. Large singly charged ions, with low charge density (e.g. $SCN^-$, $H_2PO_4^-$, $HSO_4^-$, $HCO_3^-$, $I^-$ $Cl^-$, $NO_3^-$, $NH_4^+$, $Cs^+$, $K^+$, $(NH_2)_3C^+$ (guanidinium) and $(CH_3)_4N^+$ (tetramethylammonium) ions; exhibiting weaker interactions with water than water with itself and thus interfering little in the hydrogen bonding of the surrounding water), are chaotropes whereas small or multiply-charged ions, with high charge density, are kosmotropes (e.g. $SO_4^{2-}$, $HPO_4^{2-}$, $Mg^{2+}$, $Ca^{2+}$, $Li^+$, $Na^+$, $H^+$, $OH^-$ and $HPO_4^{2-}$, exhibiting stronger interactions with water molecules than water with itself and therefore capable of breaking water-water hydrogen bonds). The radii of singly charged chaotropic ions are greater than 1.06 Å for cations and greater than 1.78 Å for anions. Thus the hydrogen bonding between water molecules is more broken in the immediate vicinity of ionic kosmotropes than ionic chaotropes. Reinforcing this conclusion, a Raman spectroscopic study of the hydrogen-bonded structure of water around the halide ions $F^-$, $Cl^-$, $Br^-$ and $I^-$ indicates that the total extent of aqueous hydrogen bonding increases with increasing ionic size and an IR study in $HDO:D_2O$ showed slow hydrogen bond reorientation around these halide ions getting slower with respect to increasing size. It is not unreasonable that a solute may strengthen some of the hydrogen bonds surrounding it (structure making; e.g. kosmotropic cations will strengthen the hydrogen bonds donated by the inner shell water molecules) whilst at the same time breaking some other hydrogen bonds (structure breaker; e.g. kosmotropic cations will weaken the hydrogen bonds accepted by the inner shell water molecules). Other factors being equal, water molecules are held more strongly by molecules with a net charge than by molecules with no net charge; as shown by the difference between zwitterionic and cationic amino acids.

Weakly hydrated ions (chaotropes, $K^+$, $Rb^+$, $Cs^+$, $Br^-$, $I^-$, guanidinium$^+$) may be "pushed" onto weakly hydrated surfaces by strong water-water interactions with the transition from strong ionic hydration to weak ionic hydration occurring where the strength of the ion-water hydration approximately equals the strength of water-water interactions in bulk solution (with $Na^+$ being borderline on the strong side and $Cl^-$ being borderline on the weak side). Neutron diffraction studies on two important chaotropes (guanidinium and thiocyanate ions) show their very poor hydration, supporting the suggestion that they preferentially interact with the protein rather than the water. In contrast to the kosmotropes, there is little significant difference between the properties of ionic and nonionc chaotropes due to the low charge density of the former.

Optimum stabilization of biological macromolecule by salt requires a mixture of a kosmotropic anion with a chaotropic cation.

Chaotropes break down the hydrogen-bonded network of water, so allowing macromolecules more structural freedom and encouraging protein extension and denaturation. Kosmotropes are stabilizing solutes which increase the order of water (such as polyhydric alcohols, trehalose, trimethylamine N-oxide, glycine betaine, ectoine, proline and various other zwitterions) whereas chaotropes create weaker hydrogen bonding, decreasing the order of water, increasing its surface tension and destabilizing macromolecular structures (such as guanidinium chloride and urea at high concentrations). Recent work has shown that urea weakens both hydrogen bonding and hydrophobic interactions but glucose acts as a kosmotrope, enhancing these properties. Thus, when urea molecules are less than optimally hydrated (about 6 - 8 moles water per mole urea) urea hydrogen bonds to itself and the protein (significantly involving the peptide links) in the absence of sufficient water, so becoming more hydrophobic and hence more able to interact with further sites on the protein, leading to localized dehydration-led denaturation. Guanidinium is a planar ion that may form weak hydrogen bonds around its edge but may establish strongly-held hydrogen-bonded ion pairs to protein carboxylates, similar to commonly found quaternary structural arginine-carboxylate "salt" links. Also, guanidinium possesses rather hydrophobic surfaces that may interact with similar protein surfaces to enable protein denaturation. Both denaturants may cause protein swelling and destructuring by sliding between hydrophobic sites and consequently dragging in hydrogen-bound water to complete the denaturation.

Generally the kosmotropic/chaotropic nature of a solute is determined from the physical bulk properties of water, often at necessarily high concentration. The change in the degree of structuring may be found, for example, using NMR or vibrational spectroscopy. Protein-stabilizing solutes (kosmotropes) increase the extent of hydrogen bonding (reducing the proton and $^{17}O$ spin-lattice relaxation times) whereas the NMR chemical shift may increase (showing weaker bonding e.g. the zwitterionic kosmotrope, trimethylamine N-oxide) or decrease (showing stronger bonding e.g. the polyhydroxy kosmotrope, trehalose). Trehalose shows both a reduction in chemical shift and relaxation time, as to a lesser extent does the protein stabilizer $(NH_4)_2SO_4$, whereas NaCl only shows a reduction in chemical shift and the protein destabilizer KSCN shows an increase in relaxation time and a reduction in chemical shift. Vibrational spectroscopy may make use of the near-IR wavelength near 5200 $cm^{-1}$ ($v_2 + v_3$ combination), which shifts towards longer wavelength (smaller wavenumber) when hydrogen bonds are stronger.

One of the most important kosmotropes is the non-reducing sugar $\alpha,\alpha$-trehalose. It should perhaps be noted that trehalose has a much more static structure than the reducing sugars, due to its lack of mutarotation, or the other common non-reducing disaccharide, sucrose, due to its lack of a furan ring.

Preferably, step (c) and/or (d) is carried out in the presence of a buffer (the "refolding buffer" and/or the "cleavage buffer"; since usually refolding and cleavage takes place simultaneously, the refolding and cleavage buffer will in most cases be the same), especially a TRIS buffer or a phosphate buffer, Brij 58, a reducing agent, especially dithiothreitol (DTT) or dithioerythritol (DTE), an ion chelating agent, especially ethylenediaminetetraacetate (EDTA), a detergent, preferably a non-ionic detergent, especially polysorbate 20, 40, 60, or 80 or octyl phenol ethoxylate; an anionic detergent, preferably a lauroyl amino acid, especially n-lauroyl-L-glutamate or lauroyl-sarcosinate; an amino acid, especially L-arginine, L-histidine or L-lysine; a carbohydrate, especially sucrose, fructose or glucose, or mixtures thereof; or mixtures of these compounds and mixtures.

If L-arginine is used as an amino acid in the cleavage buffer, 100 mM to 1 M is a preferred concentration thereof in the buffer.

A specifically preferred embodiment of the present invention employs a cleavage buffer which comprises sucrose, preferably in a concentration of 100 to 1000 mM sucrose, especially of 250 to 750 mM.

According to a preferred embodiment, the cleavage buffer has a pH of 6 to 9.5, preferably 7 to 9, especially 7.5 to 8.5.

Preferably, steps (b), (c) and/or (d) are performed in a buffer, especially a TRIS buffer or a phosphate buffer.

It is also preferred that steps (b), (c) and/or (d) are performed in a buffer containing a reducing agent, especially dithiothreitol (DTT), dithioerythritol (DTE), beta-mercaptoethanol, tris(2-carboxyethyl)phosphine (TCEP) or alpha-monothioglycerole (MTG).

It is further preferred that steps (c) and/or (d) are performed in a buffer containing an ion chelating agent, especially ethylenediaminetetraacetate (EDTA).

Preferably, step (c) is performed at a pH which does not differ from the pI of the fusion protein by more than 1, especially not more than 0.5. The pH of the buffer is therefore preferably selected near the pI of the fusion protein. However, there are also other cases wherein the pH and pI are selected independently. For example, it is also possible to perform cleavage under alkaline conditions (e.g. pH 7.5 to 9) for proteins with acidic pI (e.g. pI 5 to 6.5). This can especially be used to circumvent problems that arise out of the fact that many proteins show low solubility near their pI which could lead to aggregation.

According to another preferred embodiment, steps (c) and/or (d) are performed in the presence of a buffer comprising NaCl, preferably of 50 to 5000 mM NaCl, especially of 500 to 3000 mM NaCl.

According to a preferred embodiment, steps (c) and/or (d) are performed in the presence of a buffer comprising sucrose, DTT, NaCl, EDTA, an amino acid (e.g. L-arginine or glycine) and a detergent.

Preferred examples for the $N^{pro}$ autoprotease moiety of the fusion protein are naturally occurring versions of the $N^{pro}$ autoprotease or, preferably, deletion mutants of naturally occurring versions of the $N^{pro}$ autoprotease. Such deletions, of course, must not lead to inactivation of proteolytic activity. For example, amino acids 1 to 21 (the amino acid numbering follows the numbering of most naturally occurring $N^{pro}$ autoprotease sequences of CSFV, such as listed in Becher et al., J. Gen. Virol. 78 (1997), 1357-1366) can be deleted without affecting proteolytic activity. Other preferred variants have longer or shorter N-terminal deletions, preferably shorter deletions, e.g. variants lacking amino acids 2 to 14, 1 to 14 or 1 to 15). It is therefore preferred to use an $N^{pro}$ autoprotease lacking amino acids 1 to 21, or shorter deletions, preferably 1 to 14 or 1 to 15. These preferred autoproteases with proteolytic activity therefore start with the GluPro motif (at positions 22/23), preferably followed by a (Val/Leu)(Tyr/Phe) motif (amino acids 24 and 25 of $N^{pro}$). Another example for possible deletion without affecting proteolytic activity is amino acids 148 to 150 (e.g. ThrProArg in "EDDIE" (Achmuller et al., 2007) or GluProArg in the Alfort sequence (Becher et al., 1997)).

Sequence variations occur between the various natural isolates (see e.g. GenBank or EMBL databases); also selected mutations have been provided with improved properties (see WO 2006/113957 A; Achmuller et al., 2007; Achmuller, PhD thesis, March 2006, University of Innsbruck (AT)): For example,

- Cys112, Cys134 and Cys138 can be replaced by another amino acid residue, preferably Glu;
- His5, Lys16, Asn35, Arg53, Gly54, Arg57, Leu143, Lys145 and/or Arg150 can be replaced by another amino acid residue, preferably arginine (R) 53 with glutamic acid (E), glycine (G) 54 with aspartic acid (D), arginine (R) 57 with glutamic acid (E), and/or leucine (L) 143 with glutamine (Q);
- Val24, Ala27, Leu32, Gly54, Leu75, Ala109, Val114, Val121, Leu143, Ile155 and/or Phe158 can be replaced by another amino acid residue, preferably threonine or serine, especially alanine (A) 109, valine (V) 114, isoleucine (I) 155 and/or phenylalanine (F)158;
- Ala28, Ser71 and/or Arg150 can be replaced by another amino acid residue, preferably glutamic acid (E), phenylalanine (F) and/or with histidine (H), especially alanine (A) 28 can be replaced with glutamic acid (E), serine (S) 71 can be replaced with phenylalanine (F) and arginine (R) 150 can be replaced with histidine (H).

Preferred autoproteases can be chosen also according to the fusion partner ("protein of interest"). For example, preferred sequences are the $N^{pro}$ sequences disclosed in WO 2006/113957 A (as SEQ.ID.NOs. 1-5, 32/33, 92-98, especially SEQ.ID.NO 5 ("EDDIE")).

The present method can in principle be applied for production of any protein of interest, especially for all proteins known to be producible by the $N^{pro}$ autoprotease technique. A "protein of interest" may therefore be any protein which does - on a gene level - not naturally occur in direct 5'-3' connection with an $N^{pro}$ autoprotease. Since the method according to the present invention is suitable for large-scale manufacturing and pharmaceutical good manufacturing practice, it is preferred to produce a protein for therapeutic use in humans with the present method, preferably a human recombinant protein or a vaccination antigen.

The process parameters can be optimised for each set-up, preferably depending on the $N^{pro}$ autoprotease used and on the protein of interest to be produced.

The present invention is carried out with the $N^{pro}$ technology. This technology is disclosed e.g. in WO 01/11057 A, WO 01/11056 A, WO 2006/113957 A, WO 2006/113958 A, WO 2006/113959 A, EP 2 746 390 A1, EP 2 684 951 A1, Dürauerab et al., Sep. Sci. Technol. 45(2010), 2194-2209; Kaar et al., Biotechnol. Bioengin. 104(2009), 774-784; and Achmuller et al., Nat. Meth. 4 (2007), 1037-1043. In general terms, the $N^{pro}$ technology relates to a process for the recombinant production of a heterologous protein of interest, comprising (i) cultivation of a bacterial host cell which is transformed with an expression vector which comprises a nucleic acid molecule which codes for a fusion protein, the fusion protein comprising a first polypeptide which exhibits the autoproteolytic function of an autoprotease $N^{pro}$ of a Pestivirus, and a second polypeptide which is connected to the first polypeptide at the C-terminus of the first polypeptide in a manner such that the second polypeptide is capable of being cleaved from the fusion protein by the autoproteolytic activity of the first polypeptide, and the second polypeptide being a heterologous protein of interest, wherein cultivation occurs under conditions which cause expression of the fusion protein and formation of corresponding cytoplasmic inclusion bodies, (ii) isolation of the inclusion bodies from the host cell, (iii) solubilisation of the isolated inclusion bodies, (iv) dilution of the solubilisate to give a reaction solution in which the autoproteolytic cleavage of the heterologous protein of interest from the fusion protein is performed, and (v) isolation of the cleaved heterologous protein of interest.

This technology is suited for a large variety of proteins of interest. For the purpose of the present invention, the terms "heterologous protein", "target protein", "polypeptide of interest" or "protein of interest" (and the like) mean a polypeptide which is not naturally cleaved by an autoprotease $N^{pro}$ of a Pestivirus from a naturally occurring fusion protein or polyprotein (i.e. a polypeptide being different than the naturally following amino acids 169ff of the Pestivirus polyprotein encoding the structural Protein C and subsequent viral proteins). Examples of such heterologous proteins of interest are industrial enzymes (process enzymes) or polypeptides with pharmaceutical, in particular human pharmaceutical, activity.

Due to its autocatalytic cleavage it enables synthesis of proteins with an authentic N-terminus which is especially important for pharmaceutical applications. Furthermore, not only large proteins ("proteins of interest") but also small peptides can be stably expressed by C-terminal linking to $N^{pro}$. A high expression rate forces the fusion protein into inclusion bodies. After purification, $N^{pro}$ is refolded and cleaves itself off.

It is essential that the protein of interest to be produced by the present invention is attached C-terminally after Cys168 of the $N^{pro}$ autoprotease, because this is the cleavage site where the peptidic bond between the C-terminus of the $N^{pro}$ moiety (at Cys168) and the protein of interest is cleaved in step (d) according to the present invention.

According to a preferred embodiment, the protein of interest is a protein for therapeutic use in humans, preferably a human recombinant protein or a vaccination antigen.

Examples of preferred proteins of interest with human pharmaceutical activity are cytokines such as interleukins, for example IL-6, interferons such as leukocyte interferons, for example interferon a2B, growth factors, in particular haemopoietic or wound-healing growth factors, such as G-CSF, erythropoietin, or IGF, hormones such as human growth hormone (hGH), antibodies or vaccines. Also very short polypeptides having only 5 to 30 amino acid residues can be produced as protein of interest by the present technology. The $N^{pro}$ technology has specific advantages in an expression system making use of inclusion bodies, because the strong aggregation bias of the fused autoprotease facilitates the formation of inert inclusion bodies, almost independent of the fusion partner. Accordingly, almost any protein of interest is producible with the present system in high amounts and yields. Reports are e.g. available for expression of synthetic interferon-al; toxic gyrase inhibitor CcdB, a short 16-residue model peptide termed pep6His (SVDKLAAALEHHHHHH), human proinsulin, synthetic double domain D of staphylococcal protein A (sSpA-$D_2$), keratin-associated protein 10-4 (KRTAP10-4), synthetic green fluorescent protein variant (sGFPmut3.1), synthetic inhibitorial peptide of senescence evasion factor with N-terminal cysteine (C-sSNEVi), synthetic inhibitorial peptide of senescence evasion factor with randomized amino acid sequence with C-terminal cysteine (sSNEVscr-C); recombinant human monocyte chemoattractant protein 1 (rhMCP-1). So far, the only limitations with respect to high yields have been suspected for chaperones and proteins with comparable properties of supporting protein folding. Such proteins as fusion partners could suppress the aggregation bias of an $N^{pro}$ molecule, leading to lower yields due to less aggregation. Nevertheless, the present technology can even be applied for expressing such proteins counter-acting aggregation.

The fusion protein according to the present invention can additionally contain auxiliary sequences, such as affinity tags or refolding aid moieties; it may also contain more than one protein of interest (it can e.g. contain two or three or four or even more proteins of interest which may be separated from each other at a later stage or even at the same stage as the cleavage by the $N^{pro}$ autoprotease) .

The fusion protein according to the present invention is usually encoded by an expression vector encoding for a fusion protein comprising an $N^{pro}$ autoprotease and the protein of interest. In the expression vector to be employed in the process according to the present invention, the fusion polypeptide is operably linked to at least one expression control sequence. Expression control sequences are, in particular, promoters (such as the lac, tac, T3, T7, trp, gac, vhb, lambda pL or phoA promoter), ribosome binding sites (for example natural ribosome binding sites which belong to the abovementioned promoters, cro or synthetic ribosome binding sites), or transcription terminators (for example rrnB T1T2 or bla).

The vector may also contain sequences encoding fusion domains, as described below, that are present at the N-terminal end of the fusion polypeptide (= fusion protein) and that are required for its binding to the affinity chromatography system, e.g. polyamino acids like polylysine or, for immunoaffinity chromatogography, so-called "epitope tags", which are usually short peptide sequences for which a specific antibody is available. Well known epitope tags for which specific monoclonal antibodies are readily available include FLAG, influenza virus haemagglutinin (HA), and c-myc tags.

In a preferred embodiment of the present invention, the expression vector is a plasmid.

The present invention also applies a host cell, preferably a prokaryotic host cell, especially an E. coli host cell, containing an expression vector according to the present invention. The transformed bacterial host cell, i.e. the expression strain, is cultivated in accordance with microbiological practice known per se. The host strain is generally brought up starting from a single colony on a nutrient medium, but it is also possible to employ cryo-preserved cell suspensions (cell banks). The strain is generally cultivated in a multistage process in order to obtain sufficient biomass for further use.

On a small scale, this can take place in shaken flasks, it being possible in most cases to employ a complex medium (for example LB broth). However, it is also possible to use defined media (for example citrate medium). Since in the preferred embodiment of the present invention it is intended that the expressed fusion polypeptide is in the form of insoluble inclusion bodies, the culture will in these cases be carried out at relatively high temperature (for example 30°C or 37°C). Inducible systems are particularly suitable for producing inclusion bodies (for example with the trp, lac, tac or phoA promoter).

On a larger scale, the multistage system consists of a plurality of bioreactors (fermenters), it being preferred to employ defined nutrient media. In addition, it is possible greatly to increase biomass and product formation by metering in particular nutrients (fed batch). Otherwise, the process is analogous to the shaken flask. In the process according to the present invention, the inclusion bodies are isolated from the host cell in a manner known per se. For example, after the fermentation has taken place, the host cells are harvested by centrifugation, micro filtration, flocculation or a combination thereof, preferably by centrifugation. The wet cell mass is disintegrated by mechanical, chemical or physical means such as high pressure homogenizer, beads mills, French press, Hughes press, osmotic shock, detergents, enzymatic lysis or a combination thereof. Preferably, disruption of the cells takes place by high pressure homogenization. In the preferred embodiment where the recombinant fusion polypeptide is deposited as inclusion bodies, the inclusion bodies can be obtained for example by means of high-pressure dispersion or, preferably, by a simple centrifugation at low rotor speed. The inclusion bodies are separated by centrifugation or microfiltration or a combination thereof. The purity in relation to the desired polypeptide of interest can then be improved by multiple resuspension of the inclusion bodies in various buffers, for example in the presence of NaCl (for example 0.5 to 1.0 M) and/or detergent (for example Triton X-100). Preferably the purity of the inclusion body preparation is improved by several washing steps with various buffers (e.g. 0.5 % Deoxycholate followed by two times 1 M NaCl solution and finally distilled water). This usually results in removal of most of the foreign polypeptides from the inclusion bodies.

The present invention is further described by the following examples and the drawing figures, yet without being restricted thereto.

Figure 1: Renaturation of fusion protein 1 with and without chaotropes.
Figure 2: Renaturation of fusion protein 1 in dependence of the NLG concentration in the renaturation batch.
Figure 3: Kinetic of renaturation of fusion protein 1 with and without chaotropes.
Figure 4: Renaturation of fusion protein 1 in dependence of the NLS concentration and L-Arginine in the renaturation batch.
Figure 5: Renaturation of fusion protein 1 in dependence of the residual NLS concentration and the used renaturation buffer.
Figure 6: Renaturation of fusion protein 2 in dependence of the NLS, urea and fusion protein in the renaturation batch.

Examples

Materials and methods

Results

This summary describes the comparison of the renaturation of two different $N^{pro}$ fusion proteins in chaotrope and detergent containing buffer.

The solubilization of $N^{pro}$ fusion protein containing inclusion bodies in chaotrope (e.g. urea and guanidine hydrochloride) containing buffers and the sequential renaturation by rapid dilution is a common process in the production of recombinant therapeutic proteins. Here we show the solubilization and renaturation of two different $N^{pro}$ fusion proteins in detergent (e.g. N-Lauroyl sarcosinate and N-Lauroyl-glutamate) containing buffers.

**Materials and Methods**

All used chemicals were of Ph. Eur. Grade and were purchased by known suppliers.

The inclusion bodies from high cell density cultures of E. *coli* were extracted from the bacteria by French press and continuous centrifugation. The Inclusion bodies were washed with purified water. One part of the pre-diluted inclusion were mixed with two parts of the respective solubilization buffer and stirred at room temperature for 30 minutes. Afterwards one part of the solubilized inclusion bodies was mixed with four parts of renaturation buffer. The renaturation batch for fusion protein 1 was further stirred at 2-8 °C and the renaturation batch of fusion protein 2 was stirred at room temperature.

**Chemicals:**

All used substances were Ph. Eur. grade or of comparable quality. The buffers were prepared with purified and de-ionized water.

Substance : Supplier
1, 4-Dithiotreitol (DTT): C.F.M. Tropitzsch

2-Amino-2-(hydroxymethyl) propane-1,3-diol (Tris): Angus Chemie
3-(N-Morpholino)-Propanesulfonacid-sodium salt: Sigma-Aldrich 3-(N, N-Dimethylpalmitylammonio)propanesulfonat (Zwittergent 3-14): Sigma-Aldrich
Acetic Acid (80%): Merck
Ethylendiaminetetraacedic acid, 2 Na (EDTA): Merck
Guanidine hydrochloride: Sigma-Aldrich
Hydrochloride acid (HCL): Merck
L-Arginine hydrochloride: Ajinomoto
Polyethylene glycol hexadecyl ether (Brij 58): Sigma-Aldrich
Polysorbate 80 : BioRad
N-lauroyl glutame: Ajinomoto
Sodium N- lauroyl sarcosinate : Sigma-Aldrich
Sodium chloride (NaCl): Merck/Baker
Sucrose: Suedzucker AG
Urea: Merck

**Analytical methods:**

The analytical determination of the content of fusion protein, cleaved $N^{pro}$ and cleaved model protein was performed on a HPLC system with Autosampler and multiple wavelength detector (Agilent Technologies, Santa Clara, USA). The determination of contents of model protein 1 was performed with a Zorbax 300SB-C3 (Agilent Technologies, Santa Clara, USA) column with a bed height of 15 cm and a diameter of 4.6 mm. The particle diameter was 3.5 μm and the pore size 300 Å. The samples were diluted with sample dilution buffer (100 mM MOPS, 7 M guanidine hydrochloride, 2% (w/v) Zwittergent 3-14, 130 mM DTT, pH 7.0) to a target concentration of 150 μg/mL. The measurements were performed with a constant linear flow of 1.5 mL/min at 60 °C. The determination of contents of model protein 2 was performed with a Tosoh TSK Super Octyl (Tosoh Biosciences, Tokyo, Japan) column with a bed height of 10 cm and a diameter of 4.6 mm. The particle diameter was 2 μm. The samples were diluted with sample dilution buffer (50 mM Tris, 7 M guanidine hydrochloride, 0.5% (w/v) Polysorbate 80, 100 mM DTT, pH 8.0) to a target concentration of 225 μg/mL. The measurements were performed with a constant linear flow of 1.1 mL/min at 50 °C. The determination of the amount of released/cleaved off fusion partner was determined by ion paired reversed phase high performance chromatography. A five point calibration curve (peak area vs. measured concentration) was performed with the purified fusions partner. The cleavage yield was determined by the following equation:

$$Y = \frac{c_{fp,24h}}{c_{FP} \cdot x_{fp}} \cdot 100,$$

with

$C_{fp,24h}$ Concentration of the fusion partner after 24 h refolding [mg/mL]

$c_{FP}$ Concentration of the fusion protein at refolding start [mg/mL]

$X_{fp}$ Mass fraction of the fusion partner in the fusion protein, calculated according to

$$x_{fp} = \frac{MW_{FP} - MW_{N^{Pro}}}{MW_{FP}},$$

with

MWFP Molecular weight of the fusion protein [Da]

$MW_N^{Pro}$ Molecular weight of $N^{pro}$ moiety of the fusion protein [Da]

The buffers used are listed in Table 1.

Table 1: Buffers used for solubilization and renaturation of the used $N^{pro}$ fusion proteins

| Buffer | Composition |
|---|---|
| Solubilization 1 | 75 mM Tris, 4.5 M urea, 37.5 mM DTT, pH 7.9 |
| Solubilization 2 | 75 mM sodium phosphate, 3 % (w/v) N-lauroyl glutamate, 37.5 mM DTT, pH 7.9 |
| Solubilization 3 | 75 mM Tris, 4.5 M urea, 3 % (w/v) N-lauroyl glutamate , 37.5 mM DTT, pH 7.9 |
| Renaturation 1 (reference) | 0.625 M L-Arginine, 50 mM Tris, 25 mM DTT, 1.25 M NaCl, 6.25 mM EDTA, 0.00625 % (w/v) Brij58, 0.625 M Sucrose, pH 8.0 |
| Renaturation 2 (+urea) | 600 mM Urea, 0.625 M L-Arginine, 50 mM Tris, 25 mM DTT, 1.25 M NaCl, 6.25 mM EDTA, 0.00625 % (w/v) Brij58, 0.625 M Sucrose, pH 8.0 |
| Renaturation 3 (+NLG) | 0.4% (w/v) N- lauroyl glutamate, 0.625 M L-Arginine, 50 mM Tris, 25 mM DTT, 1.25 M NaCl, 6,25 mM EDTA, 0.00625 % (w/v) Brij58, 0.625 M Sucrose, pH 8.0 |
| Renaturation 4 | 50 mM Tris, 630 mM NaCl, 940 mM sucrose, 0.012% (w/v) Brij 58, 20 mM DTT, pH 8.0; |
| Renaturation 5 | 50 mM Tris, 20 mM DTT, 1.25 M NaCl, 6.25 mM Na-EDTA, 0.00625% (w/v) Brij 58, 625 mM sucrose, pH 8.0 |

**Results:**

**Fusion protein 1**

The fusion protein used in this example (model protein 1) consists of 338 amino acids. The 6-His-NPro-EDDIE-q15 moiety is 161 amino acids and the fusion partner consists of 177 amino acids. A 6-His tag was fused to the N-terminus of the $N^{pro}$ to enable the purification with a metal chelate affinity chromatography. Based on the amino acid sequence the isoelectric points are determined with 5.92 and 4.99 for the fusion protein and the fusion partner. The molecular masses are calculated with 18350.6 Da for 6-His-NPro-EDDIE-q15, 18893.2 Da for the fusion partner and 37243.8 Da for the fusion protein. The fusion partner has three cysteines (Cys71, Cys89 and Cys122). Cys71 and Cys89 form one intramolecular disulfide bridge. A false bridging with the Cys 122 was not shown.

The renaturation yield in the buffer even containing urea is the reference for the other determined solubilization and renaturation approaches. With additional urea in the renaturation buffer the cleavage yield decreases up to 15%. The higher chaotrope concentration inhibits the folding reaction. Additional NLG in the renaturation buffer lifts the renaturation yield up to 15%. The renaturation after solubilization of the inclusion bodies in NLG containing buffer achieved in comparison to the urea based renaturation of fusion protein 1 up to relatively 65% higher yields. The ability of NLG to stabilize the partial and unfolded fusion protein monomers is significant higher compared to urea. The positive effect of NLG during refolding was slightly decreased through the addition of additional urea and NLG in the renaturation buffer. The combined solubilization with urea and NLG resulted in lower yields than the NLG based renaturation.

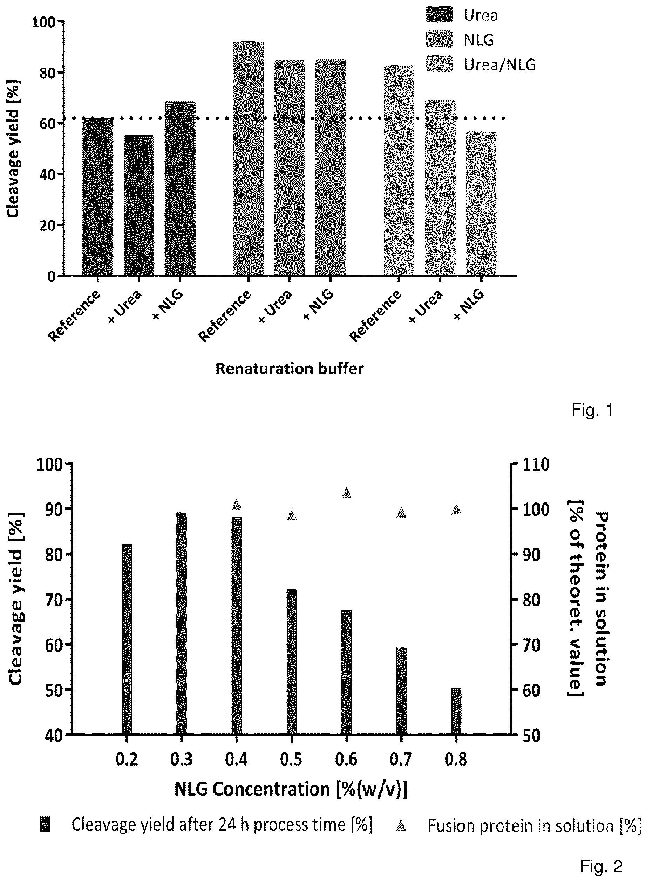

Figure 2 shows the results of the solubilization of the inclusion bodies and the renaturation of fusion protein 1 in renaturation buffer 1 with different residual concentrations of NLG.

The solubilization capability of NLG is fully given above the critical micelle concentration of the detergent. With increasing residual NLG concentration in the renaturation batch the refolding yield decreases. For the renaturation of fusion protein 1 an optimum for the residual NLG concentration was determined between 0.3 and 0.4% (w/v). In this concentration range the full ability of solubilizing of the inclusion bodies is given.

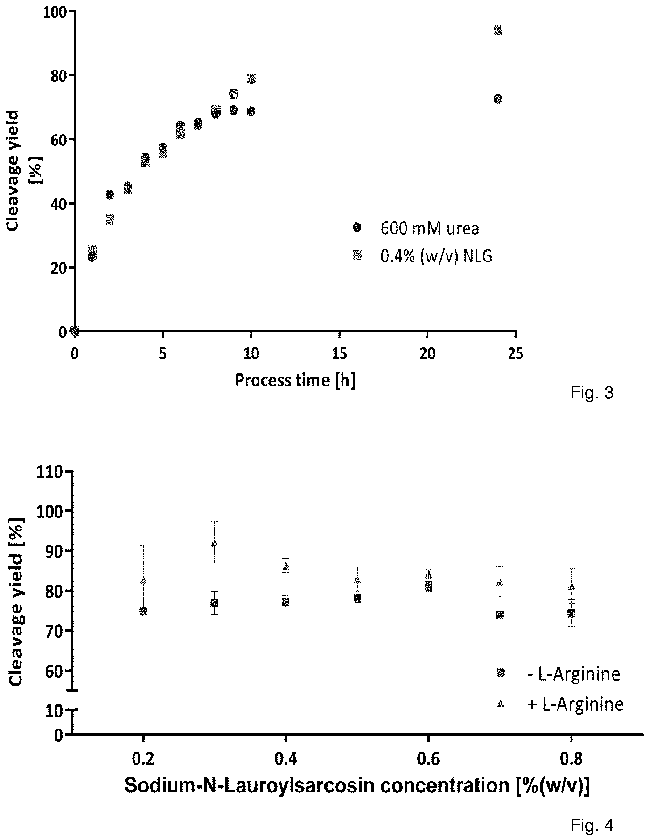

Figure 3 shows the comparison of the cleavage kinetics of the chaotrope and detergent based renaturation of fusion protein 1 in renaturation buffer 1 at a fusions protein concentration of 4 mg/mL. The solubilization was done in solubilization buffer 1 and solubilization buffer 2.

The renaturation kinetic proceeds equal in the first 8 hours of the renaturation process. Afterwards the kinetics diverges. The renaturation reaction in the urea based renaturation was finished but the refolding in the NLG containing batch continued. At the end of the monitored time range (24 h) a yield of 72% and 94% was reached for the urea and NLG based renaturation of fusion protein 1.

A second examined detergent for the chaotrope free renaturation of $N^{pro}$ fusion proteins was NLS. The results for the renaturation in dependence of the residual NLS concentration and L-Arginine in the renaturation buffer are shown in Figure 4. The solubilization of the inclusion bodies was performed with an adapted solubilization buffer 1, NLG was exchanged against NLS. The renaturation was performed with renaturation buffer 1, with and without the given concentration of L-Arginine

The renaturation yields are dependent of L-Arginine in the renaturation buffer. With L-Arginine in the renaturation buffer an up to 20% higher yield was obtained. With increased residual NLS concentration no decrease in refolding yield was observed. All in one the renaturation yields were in the same range than for the NLG based renaturation of fusion protein 1.

**Fusion protein 2**

The fusion protein used in this example (model protein 2) consists of 269 amino acids. The NPro-EDDIE-q15 moiety has 155 amino acids and the fusion partner consists of 102 amino acids. Based on the amino acid sequence the isoelectric points were calculated with 8.51 and 9.35 for the fusion protein and the fusion partner. The molecular masses were calculated with 17545.4 Da for Npro-EDDIEq15, 11232.8 Da for the fusion partner and 28760.5 Da for the fusion protein. In comparison to the naturally occurring variant of the fusion partner 33 amino acids are attached to its C-terminus. The sequence of the fusion partner contains 6 cysteines, which enable three disulfide bridges.

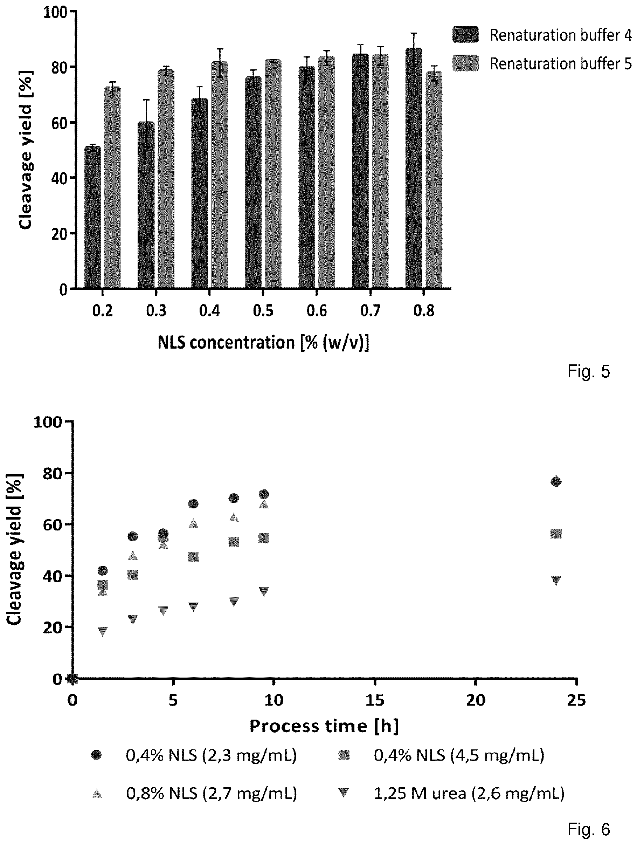

Figure 5 shows the results for the renaturation of fusion protein 2 for the renaturation buffer and NLS concentration dependent renaturation.

The yield by using the renaturation buffer 4 is significant dependent of the residual NLS concentration in the renaturation batch. The overall yield raises from 50% at 0.2% (w/v) to 83% with 0.8% (w/v) residual NLS. In renaturation buffer no such dependence f the residual NLS concentration could be observed. Slightly lower yields were obtained at the two lowest determined NLS concentrations.

Figure 6 shows the kinetics for the renaturation of fusion protein 2 at selected protein concentrations in the NLS and urea based renaturation in renaturation buffer 4.

The kinetics of the renaturation is showing three main influences for the renaturation.

1. The NLS based renaturation is at equal protein concentrations independent of the used residual concentration of NLS in the refolding batch
2. The NLS based renaturation has a high leverage dependence of the fusion protein concentration
3. The NLS based renaturation achieves at equal fusion protein concentrations significant higher yields compared to the urea based renaturation

## Claims

1. Method for producing a recombinant protein of interest, **characterised in** by the following steps:

   (a) providing a fusion protein comprising an N$^{pro}$ autoprotease moiety and a protein of interest moiety in inclusion bodies,
   (b) solubilising the fusion protein in the inclusion bodies by subjecting the inclusion bodies to a solubilisation buffer containing a detergent and wherein the solubilisation buffer contains no chaotropes or chaotropes in a concentration corresponding to less than 1.5 M urea, wherein the detergent in the solubilisation buffer is a lauroyl amino acid, especially lauroyl-L-glutamate, lauroyl-sarcosinate, or mixtures thereof;
   (c) refolding the solubilised fusion protein, and
   (d) allowing the fusion protein to be cleaved by the N$^{pro}$ autoprotease moiety under kosmotropic conditions, wherein the recombinant protein of interest is cleaved from the fusion protein, and
   (e) recovering the protein of interest.

2. Method according to claim 1, **characterized in that** the inclusion bodies were generated in a recombinant production system, preferably in a prokaryotic host cell, especially in E. coli host cells.

3. Method according to claim 1 or 2, **characterized in that** the detergent is contained in the solubilisation buffer in a concentration of 0.2 to 15 % (w/v), preferably of 0.3 to 10 % (w/v), especially of 0.4 to 5 % (w/v).

4. Method according to any one of claims 1 to 3, **characterized in that** steps (c) and/or (d) are performed at kosmotropic conditions that correspond to a urea concentration of 0 to 1.5 M, preferably from 0.2 to 1 M, especially from 0.4 to 0.8 M.

5. Method according to any one of claims 1 to 4, **characterized in that** steps (b), (c) and/or (d) are performed in a buffer, especially a TRIS buffer or a phosphate buffer.

6. Method according to any one of claims 1 to 5, **characterized in that** steps (b), (c) and/or (d) are performed in a buffer containing a reducing agent, especially dithiothreitol (DTT), alpha-monothioglycerole (MTG), beta-mercaptoethanol ($\beta$-ME), tris(2-carboxyethyl)phosphine (TCEP) or dithioerythritol (DTE).

7. Method according to any one of claims 1 to 6, **characterized in that** steps (c) and/or (d) are performed in a buffer containing an ion chelating agent, especially ethylenediaminetetraacetate (EDTA).

8. Method according to any one of claims 1 to 7, **characterized in that** steps (c) and/or (d) are performed in a buffer containing a detergent, preferably a non-ionic detergent, especially polysorbate 20, 40, 60, or 80 or octyl phenol ethoxylate; Brij 58, or an anionic detergent such as lauroyl amino acid, especially lauroyl-L-glutamate, or mixtures thereof; and/or an amino acid, especially L-arginine, L-histidine, L-lysine or mixtures thereof; and/or a carbohydrate, especially sucrose, fructose, glucose or mixtures thereof; or mixtures thereof.

9. Method according to any one of claims 1 to 8, **characterized in that** steps (c) and/or (d) are performed in a buffer with a pH of 6 to 9.5, preferably 7 to 9, especially 7.5 to 8.5.

10. Method according to any one of claims 1 to 9, **characterized in that** the protein of interest is a protein for therapeutic use in humans, preferably a human recombinant protein or a vaccination antigen.

11. Method according to any one of claims 1 to 10, **characterized in that** steps (c) and/or (d) are performed in the presence of a buffer comprising NaCl, preferably of 50 to 5000 mM NaCl, especially of 500 to 3000 mM NaCl.

12. Method according to any one of claims 1 to 11, **characterized in that** that steps (c) and/or (d) are performed in the presence of a buffer comprising sucrose, DTT, NaCl, EDTA and a detergent.

## Patentansprüche

1. **1.** Verfahren zur Herstellung eines rekombinanten Proteins von Interesse, **gekennzeichnet durch** die folgenden Schritte:

    (a) Bereitstellen eines Fusionsproteins, das einen N$^{pro}$-Autoprotease-Anteil und einen Protein-von-Interesse-Anteil in Einschlusskörperchen umfasst,
    (b) Solubilisieren des Fusionsproteins in den Einschlusskörperchen durch Unterziehen der Einschlusskörperchen einem Solubilisierungspuffer, der ein Detergens enthält, und wobei der Solubilisierungspuffer keine Chaotrope oder Chaotrope in einer Konzentration entsprechend weniger als 1,5 M Harnstoff enthält, wobei das Detergens in dem Solubilisierungspuffer eine Lauroylaminosäure, insbesondere Lauroyl-L-glutamat, Lauroylsarcosinat oder Mischungen daraus, ist;
    (c) Rückfalten des solubilisierten Fusionsproteins, und
    (d) Ermöglichen der Spaltung des Fusionsproteins durch den N$^{pro}$-Autoprotease-Anteil unter kosmotropen Bedingungen, wobei das rekombinante Protein von Interesse von dem Fusionsprotein abgespalten wird, und
    (e) Rückgewinnen des Proteins von Interesse.

2. **2.** Verfahren gemäß Anspruch 1, **dadurch gekennzeichnet, dass** die Einschlusskörperchen in einem rekombinanten Produktionssystem, vorzugsweise in einer prokaryotischen Wirtszelle, insbesondere in E. coli-Wirtszellen, erzeugt wurden.

3. **3.** Verfahren gemäß Anspruch 1 oder 2, **dadurch gekennzeichnet, dass** das Detergens in dem Solubilisierungspuffer in einer Konzentration von 0,2 bis 15 % (w/v), vorzugsweise von 0,3 bis 10 % (w/v), insbesondere von 0,4 bis 5 % (w/v), enthalten ist.

4. **4.** Verfahren gemäß einem der Ansprüche 1 bis 3, **dadurch gekennzeichnet, dass** die Schritte (c) und/oder (d) bei kosmotropen Bedingungen, die einer Harnstoffkonzentration von 0 bis 1,5 M, vorzugsweise von 0,2 bis 1 M, insbesondere von 0,4 bis 0,8 M entsprechen, durchgeführt werden.

5. **5.** Verfahren gemäß einem der Ansprüche 1 bis 4, **dadurch gekennzeichnet, dass** die Schritte (b), (c) und/oder (d) in einem Puffer, insbesondere einem TRIS-Puffer oder einem Phosphatpuffer, vorgenommen werden.

6. **6.** Verfahren gemäß einem der Ansprüche 1 bis 5, **dadurch gekennzeichnet, dass** die Schritte (b), (c) und/oder (d) in einem Puffer, enthaltend ein Reduktionsmittel, insbesondere Dithiothreitol (DTT), Alpha-Monothioglycerol (MTG), Beta-Mercaptoethanol ($\beta$-ME), Tris(2-carboxyethyl)phosphin (TCEP) oder Dithioerythritol (DTE), vorgenommen werden.

7. **7.** Verfahren gemäß einem der Ansprüche 1 bis 6, **dadurch gekennzeichnet, dass** die Schritte (c) und/oder (d) in einem Puffer, der einen Ionenchelatbildner, insbesondere Ethylendiamintetraacetat (EDTA), enthält, vorgenommen werden.

8. **8.** Verfahren gemäß einem der Ansprüche 1 bis 7, **dadurch gekennzeichnet, dass** die Schritte (c) und/oder (d) in einem Puffer vorgenommen werden, der ein Detergens, vorzugsweise ein nicht-ionisches Detergens, insbesondere Polysorbat 20, 40, 60 oder 80 oder Octylphenolethoxylat; Brij 58, oder ein anorganisches Detergens, wie Lauroylaminosäure, insbesondere Lauroyl-L-glutamat, oder Mischungen daraus; und/oder eine Aminosäure, insbesondere L-Arginin, L-Histidin, L-Lysin oder Mischungen daraus; und/oder ein Kohlenhydrat, insbesondere Sukrose, Fruktose, Glukose oder Mischungen daraus; oder Gemische aus diesen enthält.

9. **9.** Verfahren gemäß einem der Ansprüche 1 bis 8, **dadurch gekennzeichnet, dass** die Schritte (c) und/oder (d) in einem Puffer mit einem pH von 6 bis 9,5, vorzugsweise von 7 bis 9, insbesondere von 7,5 bis 8,5, vorgenommen werden.

10. **10.** Verfahren gemäß einem der Ansprüche 1 bis 9, **dadurch gekennzeichnet, dass** das Protein von Interesse ein Protein zur therapeutischen Anwendung beim Menschen, vorzugsweise ein humanes rekombinantes Protein oder ein Vakzinationsantigen, ist.

11. **11.** Verfahren gemäß einem der Ansprüche 1 bis 10, **dadurch gekennzeichnet, dass** die Schritte (c) und/oder (d) in Gegenwart eines Puffers, umfassend NaCl, vorzugsweise 50 bis 5000 mM NaCl, insbesondere 500 bis 3000 mM NaCl, vorgenommen werden.

12. Verfahren gemäß einem der Ansprüche 1 bis 11, **dadurch gekennzeichnet, dass** die Schritte (c) und/oder (d) in Gegenwart eines Puffers, umfassend Sukrose, DTT, NaCl, EDTA und ein Detergens, vorgenommen werden.

## Revendications

1. Procédé de production d'une protéine recombinante d'intérêt, **caractérisé par** les étapes suivantes :

   (a) fourniture d'une protéine de fusion comprenant un groupement d'autoprotéase $N^{pro}$ et un groupement de protéine d'intérêt dans des corps d'inclusion,
   (b) solubilisation de la protéine de fusion dans les corps d'inclusion en soumettant les corps d'inclusion à un tampon de solubilisation contenant un détergent et où le tampon de solubilisation ne contient pas de chaotropes ou des chaotropes à une concentration correspondant à moins de 1,5 M d'urée, où le détergent dans le tampon de solubilisation est un lauroyl-acide aminé, en particulier le lauroyl-L-glutamate, le lauroyl-sarcosinate, ou leurs mélanges ;
   (c) le repliement de la protéine de fusion solubilisée, et
   (d) le fait de laisser la protéine de fusion être clivée par le groupement d'autoprotéase $N^{pro}$ dans des conditions cosmotropiques, où la protéine recombinante d'intérêt est clivée de la protéine de fusion, et
   (e) la récupération de la protéine d'intérêt.

2. Procédé selon la revendication 1, **caractérisé en ce que** les corps d'inclusion ont été produits dans un système de production recombinant, de préférence dans une cellule hôte procaryote, en particulier dans des cellules hôtes d'E. coli.

3. Procédé selon la revendication 1 ou 2, **caractérisé en ce que** le détergent est contenu dans le tampon de solubilisation à une concentration de 0,2 à 15 % (p/v), de préférence de 0,3 à 10 % (p/v), en particulier de 0,4 à 5 % (p/v).

4. Procédé selon l'une quelconque des revendications 1 à 3, **caractérisé en ce que** les étapes (c) et/ou (d) sont effectuées dans des conditions cosmotropiques qui correspondent à une concentration d'urée de 0 à 1,5 M, de préférence de 0,2 à 1 M, en particulier de 0,4 à 0,8 M.

5. Procédé selon l'une quelconque des revendications 1 à 4, **caractérisé en ce que** les étapes (b), (c) et/ou (d) sont effectuées dans un tampon, en particulier un tampon TRIS ou un tampon au phosphate.

6. Procédé selon l'une quelconque des revendications 1 à 5, **caractérisé en ce que** les étapes (b), (c) et/ou (d) sont effectuées dans un tampon contenant un agent réducteur, en particulier le dithiothréitol (DTT), l'alpha-monothioglycérol (MTG), le béta-mercaptoéthanol (β-ME), la tris(2-carboxyéthy)phosphine (TCEP) ou le dithioérythritol (DTE).

7. Procédé selon l'une quelconque des revendications 1 à 6, **caractérisé en ce que** les étapes (c) et/ou (d) sont effectuées dans un tampon contenant un agent chélatant ionique, en particulier l'éthylènediaminetétraacétate (EDTA).

8. Procédé selon l'une quelconque des revendications 1 à 7, **caractérisé en ce que** les étapes (c) et/ou (d) sont effectuées dans un tampon contenant un détergent, de préférence un détergent non ionique, en particulier le polysorbate 20, 40, 60 ou 80 ou l'octylphénoléthoxylate ; le Brij 58, ou un détergent anionique tel qu'un lauroyl-acide aminé, en particulier le lauroyl-L-glutamate, ou leurs mélanges ; et/ou un acide aminé, en particulier la L-arginine, la L-histidine, la L-lysine ou leurs mélanges ; et/ou un glucide, en particulier le saccharose, le fructose, le glucose ou leurs mélanges ; ou leurs mélanges.

9. Procédé selon l'une quelconque des revendications 1 à 8, **caractérisé en ce que** les étapes (c) et/ou (d) sont effectuées dans un tampon avec un pH de 6 à 9,5, de préférence de 7 à 9, en particulier de 7,5 à 8,5.

10. Procédé selon l'une quelconque des revendications 1 à 9, **caractérisé en ce que** la protéine d'intérêt est une protéine pour un usage thérapeutique chez les êtres humains, de préférence une protéine recombinante humaine ou un antigène de vaccination.

**11.** Procédé selon l'une quelconque des revendications 1 à 10, **caractérisé en ce que** les étapes (c) et/ou (d) sont effectuées en présence d'un tampon comprenant du NaCl, de préférence de 50 à 5000 mM de NaCl, en particulier de 500 à 3000 mM de NaCl.

**12.** Procédé selon l'une quelconque des revendications 1 à 11, **caractérisé en ce que** les étapes (c) et/ou (d) sont effectuées en présence d'un tampon comprenant du saccharose, du DTT, du NaCl, de l'EDTA et un détergent.

Urea
NLG
Urea/NLG
100
80
60
40
20
0
Cleavage yield [%]
Reference
+ Urea
+ NLG
Reference
+ Urea
+ NLG
Reference
+ Urea
+ NLG
Renaturation buffer

Fig. 1

100
90
80
70
60
50
40
Cleavage yield [%]
110
100
90
80
70
60
50
Protein in solution [% of theoret. value]
0.2
0.3
0.4
0.5
0.6
0.7
0.8
NLG Concentration [%(w/v)]
Cleavage yield after 24 h process time [%]
Fusion protein in solution [%]

Fig. 2

Cleavage yield [%]
100
80
60
40
20
0
0
5
10
15
20
25
Process time [h]
600 mM urea
0.4% (w/v) NLG

Fig. 3

Cleavage yield [%]
110
100
90
80
70
60
10
0
0.2
0.4
0.6
0.8
Sodium-N-Lauroylsarcosin concentration [%(w/v)]
- L-Arginine
+ L-Arginine

Fig. 4

Cleavage yield [%]
100
80
60
40
20
0
0.2
0.3
0.4
0.5
0.6
0.7
0.8
NLS concentration [% (w/v)]
Renaturation buffer 4
Renaturation buffer 5

Fig. 5

Cleavage yield [%]
100
80
60
40
20
0
0
5
10
15
20
25
Process time [h]
0,4% NLS (2,3 mg/mL)
0,4% NLS (4,5 mg/mL)
0,8% NLS (2,7 mg/mL)
1,25 M urea (2,6 mg/mL)

Fig. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 2746390 A1
- WO 2006113957 A
- WO 0111057 A
- WO 0111056 A
- WO 2006113958 A
- WO 2006113959 A
- EP 2684951 A1
- EP 15154320 A


### Non-patent literature cited in the description


- **VALLEJO et al.** *Microb. Cell Fact.,* 2004, vol. 3, 11
- **JUNGBAUER et al.** *J. Biotech.,* 2007, vol. 128, 587-596
- **DURAURAB et al.** *Separation Science and technology,* 2010, 2194-2209
- **ACHMULLER et al.** *Nat. Methods,* 2007, vol. 4, 1037-1043
- **ARIKI et al.** *J. Biochem.,* 2012, vol. 151, 27-33
- **BECHER et al.** *J. Gen. Virol.,* 1997, vol. 78, 1357-1366
- **DÜRAUERAB et al.** *Sep. Sci. Technol.,* 2010, vol. 45, 2194-2209
- **KAAR et al.** *Biotechnol. Bioengin.,* 2009, vol. 104, 774-784
- **ACHMULLER et al.** *Nat. Meth.,* 2007, vol. 4, 1037-1043